# EUROPEAN PATENT APPLICATION

(11) **EP 4 549 938 A1**
(43) Date of publication of application: **07.05.2025**
(21) Application number: 23831030.4
(22) Date of filing: 09.06.2023
(51) Int. Cl.: G01N 33/53, C07K 14/47, C07K 16/18, C12M 1/34, C12N 15/13

(54) **METHOD FOR TESTING DISEASE PROGRESSION TOWARD VASCULITIS OR TESTING RISK OF COVID-19 BECOMING SEVERE, TESTING KIT, COMPANION DIAGNOSTIC AGENT, AND TESTING MARKER**

(30) Priority: 30.06.2022 JP 2022105805
(71) Applicant: A-Clip Institute, Co., Ltd., Chiba-City, Chiba 260-0842 (JP)
(72) Inventor: SUZUKI, Kazuo, Chiba-shi, Chiba 260-8670 (JP); FURUTA, Shunsuke, Chiba-shi, Chiba 260-8677 (JP); EBATA, Ryota, Chiba-shi, Chiba 260-8677 (JP); IGARI, Hidetoshi, Chiba-shi, Chiba 260-8677 (JP); KAMEOKA, Yosuke, Chiba-City, Chiba 260-0842 (JP)
(74) Representative: Crow, Martin
(86) International application number: PCT/JP2023/021487
(87) International publication number: WO 2024/004580

(57) **Abstract**

[Problem] Provided are a new marker of disease activity useful for testing a diseased state of vasculitis or an exacerbation risk of COVID-19, and a means for testing a diseased state of vasculitis or an exacerbation risk of COVID-19 using the marker.

[Solution] According to an aspect of the present invention, there are provided a method for testing a diseased state of vasculitis and a method for testing an exacerbation risk of COVID-19, the method including: detecting or quantifying, in a blood sample collected from a subject,
(1) a No. 1 APOA2-like protein that causes an antigen-antibody reaction with a single chain variable region fragment consisting of an amino acid sequence set forth in SEQ ID NO: 1 and has a molecular weight of 23 to 25 kDa, and/or
(2) a No. 2 APOA2-like protein that causes an antigen-antibody reaction with a single chain variable region fragment consisting of an amino acid sequence set forth in SEQ ID NO: 1 and has a molecular weight of 16 to 20 kDa.

## Description

### TECHNICAL FIELD

The present invention relates to a method for testing a diseased state of vasculitis or an exacerbation risk of COVID-19, and a testing kit, a companion diagnostic agent, and a testing marker used therein.

### BACKGROUND ART

Autoantibodies (MPO-ANCAs (Anti-Neutrophil Cytoplasmic Antibodies)) against myeloperoxidase (MPO) in neutrophil cytoplasm are associated with refractory vasculitis such as crescent forming nephritis, microscopic polyangiitis (MPA), and eosinophilic granulomatosis with polyangiitis (EGPA). Since the titer of MPO-ANCA in the serum of patients with these diseases correlates with the activity of the disease, MPO-ANCA is used as a specific marker for these diseases for diagnosis and therapeutic determination (see, for example, Non-Patent Literature 1).

However, even when MPO-ANCA is used as a marker, the titer of MPO-ANCA in the serum does not necessarily coincide with the disease activity in some cases. This suggests that MPO-ANCA alone does not act on neutrophils to induce vasculitis or is involved after the onset of vasculitis, and there are other inducing molecules and mechanisms of vasculitis.

In recent years, the possibility that target molecules of autoantibodies are present in addition to MPO of neutrophils has been discussed. As a mechanism of onset of vasculitis and progress of the disease activity, attempts have been made to elucidate the activation mechanism of vascular endothelial cells by autoantibodies including MPO-ANCA, and it has been reported that anti-proteinase-3 autoantibodies (PR3-ANCA) of patients with granulomatosis with polyangiitis induce expression of ICAM-1 and VCAM-1, respectively (see Non-Patent Literatures 2 and 3). However, the direct effect of MPO-ANCA on vascular endothelial cells has not been reported.

As described above, at present, while there are suggestions on the presence of other factors involved in the disease activity of vasculitis in addition to MPO-ANCA clinically used for diagnosis and the like of vasculitis, the essence of such factors is not clear. As mentioned above, the diagnosis and treatment judgment by MPO-ANCA do not always fully reflect the true condition of the disease, so the identification of factors other than MPO-ANCA has the potential to provide new clinical markers that complement or replace the diagnosis by MPO-ANCA, and it is of extreme significance in terms of clinical medicine.

For Kawasaki disease, which is a vasculitis syndrome with vasculitis in a whole-body medium and small muscular artery as a main lesion, although diagnostic criteria have been established, there is currently no specific marker of disease activity. In the spread of the novel coronavirus infection (COVID-19) worldwide since 2020, direct damage by the SARS-CoV-2 virus is observed due to binding of vascular endothelial cells to the ACE2 receptor, but there are cases with exacerbation despite PCR negativity, and it is also known that vasculitis is induced by an immunological response on the host side (see Non-Patent Literatures 4 and 5). However, there is no report of markers of disease activity indicating the involvement of vasculitis as a severity factor of COVID-19.

The present inventors have found that one single chain variable region fragment (ScFv; also referred to as "VasSF") identified from the IgG genetic library of 204 clones cloned from human lymphocytes (Patent Literature 1) suppresses vasculitis at a low dose in the ANCA-related vasculitis model mouse SCG/Kj. It has also been published that the target molecule to which ScFv binds is vasculitis-associated apolipoprotein A2 (vasculitis-associated apolipoprotein A-II (VAP2)) (see Patent Literature 2 and Non-Patent Literature 6).

### Citation List

### Patent References

Patent Reference 1: JP 2016-160265 A
Patent Reference 2: WO 2018/139608 A

### Non-Patent References

Non-Patent Reference 1: Goeken JA, Antineutrophil cytoplasmic antibody-A useful serological marker for vasculitis. J. Clin. Immunol., 1991; 11: 61-74
Non-Patent Reference 2: Johnson PA, et al. Up-regulation of the endothelial cell adhesion molecule intercellular adhesion molecule-1 (ICAM-1) by autoantibodies in autoimmune vasculitis. Clin. Exp. Immunol., 1997; 108: 234-242
Non-Patent Reference 3: Mayet WJ et al., Antibodies to proteinase 3 mediate expression of vascular cell adhesion molecule-1 (VCAM-1). Clin. Exp. Immunol., 1996; 103: 25 9-267
Non-Patent Reference 4: Ackermann M et al. Pulmonary Vascular Endothelialitis, Thrombosis, and Angiogenesis in Covid-19. N. Engl. J. Med., 2020; 383(2): 120-128
Non-Patent Reference 5: Nishiga M et al., COVID-19 and cardiovascular disease: from basic mechanisms to clinical perspectives. Nature Reviews, 2020; 17(9): 543-558
Non-Patent Reference 6: Kameoka Y et al., Efficacy of a recombinant single-chain fragment variable region, VasSF, as a new drug for vasculitis. Drug Des. Devel. Ther., 2019; 13: 555-568

### SUMMARY OF INVENTION

### Technical Problem

In view of the above-described conventional techniques, an object of the present invention is to provide a new marker of disease activity useful for testing a diseased state of vasculitis or an exacerbation risk of COVID-19, and a means for testing a diseased state of vasculitis or an exacerbation risk of COVID-19 using the marker.

### Solution to Problem

The present inventors have conducted intensive studies in order to solve the above-described problems. As a result, Surprisingly, they surprisingly found that VAP2 (molecular weight = about 24 kDa), which was known to be a target molecule in the treatment of vasculitis using VasSF, was present in the serum of patients during the active phase of vasculitis-associated diseases such as ANCA-associated vasculitis, Kawasaki disease, and COVID-19 at a high level. In addition, they also found that, at the time of discharge from the hospital and during the remission phase of the disease in these patients, serum levels of proteins to which VasSF binds but which have a smaller molecular weight than VAP2 (molecular weight = approximately 17 to 19 kDa) increased in addition to a decrease in serum levels of VAP2. Based on these findings, they were able to complete the present invention.

That is, according to an aspect of the present invention, there are provided a method for testing a diseased state of vasculitis and a method for testing an exacerbation risk of COVID-19, the method including: detecting, in a blood sample collected from a subject,
(1) an apolipoprotein A2-like protein that causes an antigen-antibody reaction with a single chain variable region fragment consisting of an amino acid sequence set forth in SEQ ID NO: 1 and has a molecular weight of 23 to 25 kDa (in the present specification, this protein is referred to as "No. 1 APOA2-like protein"), and/or (2) an apolipoprotein A2-like protein that causes an antigen-antibody reaction with a single chain variable region fragment consisting of an amino acid sequence set forth in SEQ ID NO: 1 and has a molecular weight of 16 to 20 kDa (in the present specification, this protein is referred to as "No. 2 APOA2-like protein").

According to another aspect of the present invention, there is provided a testing kit for a diseased state of vasculitis or a testing kit for an exacerbation risk of COVID-19, the testing kit including a single chain variable region fragment consisting of an amino acid sequence set forth in SEQ ID NO: 1 or an anti-apolipoprotein A2 antibody.

According to still another aspect of the present invention, there is provided a companion diagnostic agent for a diseased state of vasculitis or a companion diagnostic agent for an exacerbation risk of COVID-19 used in the method according to the embodiment of the present invention described above, the companion diagnostic agent containing a single chain variable region fragment consisting of an amino acid sequence set forth in SEQ ID NO: 1 or an anti-apolipoprotein A2 antibody.

According to still another aspect of the present invention, there are provided a testing marker for a diseased state of vasculitis and a testing marker for an exacerbation risk of COVID-19, the testing marker comprising (1) a No. 1 APOA2-like protein that causes an antigen-antibody reaction with a single chain variable region fragment consisting of an amino acid sequence set forth in SEQ ID NO: 1 and has a molecular weight of 23 to 25 kDa, and/or (2) a No. 2 APOA2-like protein that causes an antigen-antibody reaction with a single chain variable region fragment consisting of an amino acid sequence set forth in SEQ ID NO: 1 and has a molecular weight of 16 to 20 kDa.

### Advantageous Effects of Invention

According to the present invention, there is provided a new marker useful for testing disease activity of vasculitis and testing an exacerbation risk of COVID-19. Based on this finding, methods and kits for testing for vasculitis susceptibility and risk of COVID-19 severity will also be provided.

### BRIEF DESCRIPTION OF DRAWINGS

Fig. 1 is a view showing the results of signal analysis of VasSF binding molecules by Western blotting method using serum samples collected from five patients with ANCA-related vasculitis (AAV) and one healthy control in Example 3.
Fig. 2 is a view showing the results of signal analysis of VasSF binding molecules using serum samples collected from one COVID-19 patient at different time points (at the time of hospital admission, at the time of ICU transfer, and at the time of hospital discharge) in Example 4.
Fig. 3 is a view showing the results of examining the relationship between a signal value of VasSF binding molecules and the disease activity of AAV for 67 specimens including 34 specimens of paired sera of an active phase and a remission phase in 17 patients with ANCA-related vasculitis (AAV) and 10 specimens of healthy controls in Example 5.
Fig. 4 is a view showing the results of examining the relationship between a signal value of VasSF binding molecules and the disease activity of COVID-19 for serum samples collected from 65 COVID-19 patients at different time points (at the time of hospital admission, at the time of ICU transfer, and at the time of hospital discharge) in Example 6.
Fig. 5 is a view showing the results of signal analysis by the same Western blotting method as in Example 3 using an anti-APOA2 antibody in Example 7.
Fig. 6 is a view showing the results of signal analysis by the same Western blotting method as in Example 4 using an anti-APOA2 antibody in Example 8.
Fig. 7 is a view showing the results of examining the relationship between a signal value of anti-APOA2 antibody binding molecules and the disease activity of COVID-19 by the same method as in Example 6 for serum samples collected from 65 COVID-19 patients at different time points (at the time of hospital admission, at the time of ICU transfer, and at the time of hospital discharge) in Example 9.
Fig. 8 is a view showing the results of signal analysis of VasSF binding molecules using serum samples collected from one pediatric patient with Kawasaki disease at different time points (Days 6, 10, and 30 from onset) in Example 10.
Fig. 9 is a view showing the results of signal analysis of VasSF binding molecules by the same method as in Example 4 using serum samples collected from five COVID-19 patients at different time points (at the time of hospital admission and at the time of hospital discharge) in Example 11.
Fig. 10A is a photograph showing the results of two-dimensional electrophoresis performed using serum of a patient in an active phase and treatment with VasSF in Example 12.
Fig. 10B is a photograph showing the results of two-dimensional electrophoresis performed using serum of a patient in a remission phase and treatment with VasSF in Example 12.

### DESCRIPTION OF EMBODIMENTS

### <<Testing marker ( marker for disease activity)>>

As described above, the present inventors have found that VAP2 (molecular weight = about 24 kDa), which was known to be a target molecule in the treatment of vasculitis with VasSF, is present at a high level in the serum of patients during the active phase of diseases with vasculitis such as ANCA-related vasculitis, Kawasaki disease, and COVID-19. Further, the present inventors have also found that the serum level of a protein which the VasSF binds to but has a smaller molecular weight (molecular weight = about 17 to 19 kDa) than the VAP2 is elevated at the time of hospital discharge and the remission state of these patents.

Based on the above findings, according to the present invention, the following markers are provided as a testing marker for a diseased state of vasculitis and a testing marker for an exacerbation risk of COVID-19:
a testing marker for a diseased state of vasculitis, comprising a No. 1 APOA2-like protein that causes an antigen-antibody reaction with a single chain variable region fragment consisting of an amino acid sequence set forth in SEQ ID NO: 1 and has a molecular weight of 23 to 25 kDa;
a testing marker for a diseased state of vasculitis, comprising a No. 2 APOA2-like protein that causes an antigen-antibody reaction with a single chain variable region fragment consisting of an amino acid sequence set forth in SEQ ID NO: 1 and has a molecular weight of 16 to 20 kDa;
a testing marker for an exacerbation risk of COVID-19, comprising a No. 1 APOA2-like protein that causes an antigen-antibody reaction with a single chain variable region fragment consisting of an amino acid sequence set forth in SEQ ID NO: 1 and has a molecular weight of 23 to 25 kDa; and
a testing marker for an exacerbation risk of COVID-19, comprising a No. 2 APOA2-like protein that causes an antigen-antibody reaction with a single chain variable region fragment consisting of an amino acid sequence set forth in SEQ ID NO: 1 and has a molecular weight of 16 to 20 kDa.

It is known that human apolipoprotein A2 (hAPOA2) is encoded by an APOA2 gene (RefSeq Accession No. NM_001643) consisting of 473 bp, and consists of 100 amino acids (RefSeq Accession No. NP_001634), and the molecular weight thereof is about 11 kDa. Therefore, both proteins as testing markers in (1) and (2) above are different molecules from human apolipoprotein A2 (hAPOA2) in terms of their molecular weight. On the other hand, all of the above proteins as testing markers cause antigen-antibody reactions with a single chain variable region fragment consisting of the amino acid sequence set forth in SEQ ID NO: 1. All of these proteins have also been confirmed to cause antigen-antibody reactions with an anti-human APOA2 antibody. This is why these proteins are referred to herein as "apolipoprotein A2 'like' proteins".

The isoelectric points (pI) of the No. 1 APOA2-like protein and the No. 2 APOA2-like protein are not particularly limited, but in a preferred embodiment, the isoelectric point (pI) of the No. 1 APOA2-like protein is preferably 6.0 to 6.6, more preferably 6.1 to 6.5, and still more preferably 6.2 to 6.4. Then, the isoelectric point (pI) of the No. 2 APOA2-like protein is preferably 5.2 to 5.8, more preferably 5.3 to 5.7, and still more preferably5.4 to 5.6. The molecular weights and isoelectric points of the No. 1 APOA2-like protein and the No. 2 APOA2-like protein can be measured by using the two-dimensional electrophoresis as described in the EXMPLES section mentioned later.

### <<Method for testing disease activity of vasculitis and method for testing exacerbation risk of COVID-19>>

According to another aspect of the present invention, there are provided a method for testing a diseased state of vasculitis and a method for testing an exacerbation risk of COVID-19, using the testing marker (marker of disease activity) according to an aspect of the present invention described above. That is, another aspect of the present invention is a method for testing a diseased state of vasculitis and a method for testing an exacerbation risk of COVID-19 (hereinafter, also collectively referred to as "testing method of the present invention"). The testing method of the present invention includes detecting or quantifying, in a blood sample collected from a subject,
(1) a No. 1 APOA2-like protein that causes an antigen-antibody reaction with a single chain variable region fragment consisting of an amino acid sequence set forth in SEQ ID NO: 1 and has a molecular weight of 23 to 25 kDa, and/or
(2) a No. 2 APOA2-like protein that causes an antigen-antibody reaction with a single chain variable region fragment consisting of an amino acid sequence set forth in SEQ ID NO: 1 and has a molecular weight of 16 to 20 kDa. In the testing method of the present invention, only either the No. 1 APOA2-like protein or the No. 2 APOA2-like protein may be detected or quantified. However, from the viewpoint of performing a more accurate testing, it is preferable to detect or quantify both of them.

According to such a testing method of the present invention, it is possible to determine, as the diseased state of vasculitis, for example, the presence or absence of affection of vasculitis, the type of the afflicted vasculitis, the degree of progress of the afflicted vasculitis, the severity of the afflicted vasculitis, the recovery state from the afflicted vasculitis, and the like, based on the results of detection or quantification of the proteins of the above (1) and/or (2). The degree of progress of vasculitis includes early, intermediate, and late stages. Then, the severity of vasculitis includes mild, moderate, severe, etc. Further, the exacerbation risk of COVID-19 includes mild, moderate, severe, etc.

The subject may have already been confirmed to suffer from vasculitis or COVID-19. The subject is preferably a subject before vasculitis or COVID-19 gets severe (that is, a subject in a mild or moderate condition), and particularly preferably a subject in a mild condition.

In the testing method of the present invention, the blood sample is not particularly limited as long as it can detect the No. 1 APOA2-like protein and the No. 2 APOA2-like protein. Examples of the blood sample include plasma and serum, and serum is preferably used.

The vasculitis to be tested by the testing method of the present invention is not particularly limited, and examples thereof include ANCA-related vasculitis such as microscopic polyangiitis (MPA), granulomatosis with polyangiitis, and eosinophilic granulomatosis with polyangitis (EGPA); Kawasaki disease; COVID-19 (Coronavirus Disease 2019; SARS-CoV-2 infection); and various kinds of vasculitis or vasculitis associated with various diseases such as agammaglobulinemia, Guillain-Barre syndrome, Wegener's granulomatosis, thrombotic thrombocytopenic purpura (TTP), IgA vasculitis, rapidly progressive glomerulonephritis, idiopathic interstitial pneumonia, idiopathic pulmonary fibrosis, sarcoidosis, diffuse panbronchiolitis, Behcet's disease, systemic lupus erythematosus (SLE), Sjogren's syndrome, Takayasu arteritis, Buerger's disease, polyarteritis nodosa, malignant rheumatoid arthritis, giant cell arteritis, anti-phospholipid antibody syndrome, scleroderma, eosinophilic fasciitis, pemphigus, nephritis, and glomerulonephritis. Among them, the testing method for a diseased state of vasculitis of the present invention is particularly useful for the testing of not only ANCA-related vasculitis and Kawasaki disease but also vasculitis associated with COVID-19.

As shown in EXAMPLES described later, the No. 1 APOA2-like protein (VAP2) is present at a high level in the serum of a patient in an active phase of vasculitis or a disease accompanied with vasculitis such as ANCA-related vasculitis, COVID-19, and Kawasaki disease, and disappears after the symptoms are alleviated. The No. 2 APOA2-like protein is not found in the serum in an active phase of the disease and is present at a high level in the serum after the symptoms are alleviated.

From these, when the concentration of the No. 1 APOA2-like protein (VAP2) in the serum of the subject is equal to or greater than a predetermined value (cut-off value), it can be determined that the subject is in an active phase of the disease or the possibility thereof is high. Further, when the subject is a patient with COVID-19, it can be determined that there is a risk that the disease gets severe or the possibility thereof is high. On the other hand, when the concentration of the No. 1 APOA2-like protein (VAP2) in the serum of the subject is less than a predetermined value (cut-off value), it can be determined that the subject is not in an active phase of the disease or the possibility thereof is low. Further, when the subject is a patient with COVID-19, it can be determined that there is no risk that the disease gets severe or the possibility thereof is low.

When the concentration of the No. 2 APOA2-like protein in the serum of the subject is equal to or greater than a predetermined value (cut-off value), it can be determined that the subject is in a remission phase or a cured state through the active phase of the disease or the possibility thereof is high. When the subject is a patient with COVID-19, it can be determined that there is no risk that the disease gets severe again or the possibility thereof is low.

Since it is difficult to uniquely define a specific value of the cut-off value for making these determinations, the cut-off value may be appropriately determined by a therapeutic person. However, if the cut-off value is too low, there is a risk that false positives will increase, and if the cutoff value is too high, there is a risk of missing patients suffering from the above diseases, for example.

In view of the above-described dynamics of the No. 1 APOA2-like protein as a marker for disease activity, it is preferable that a subject as a target of the testing method according to the present invention is a patient in an early stage after onset. Specifically, patients within 10 days are preferred, patients within 7 days are more preferred, patients within 5 days are still more preferred, patients within 3 days are particularly preferred, and patients within 1 day are most preferred, from the onset of the disease

From the viewpoint of monitoring the progress or passage of the disease activity, the detection or quantification is preferably performed a plurality of times (preferably at least 2 times, more preferably 3 times or more, still more preferably 4 times or more) at intervals of a predetermined number of days. There is no particular limit as the upper limit of the number of times of the above detection or quantification, and for example, the upper limit includes 15 times or less or 10 times or less. There is no particular limit as to the intervals of the number of days when the above detection or quantification is performed a plurality of times, and (quantification once every) one day or more is mentioned, two days or more is preferred, three days or more is still more preferred, and four days or more is particularly preferred. The upper limit value of the intervals of the number of days in the case of performing the detection or quantification a plurality of times is not particularly limited, and is, for example, once every three weeks, once every two weeks, or the like.

The testing method of the present invention may or may not include a step of collecting a serum sample from a subject.

In the testing method of the present invention, the method for detecting or quantifying the No. 1 APOA2-like protein and the No. 2 APOA2-like protein is not particularly limited, and examples thereof include assay techniques adopting enzyme immunoassay (EIA, ELISA), fluorescence enzyme immunoassay (FLEIA), chemiluminescence enzyme immunoassay (CLEIA), chemiluminescence immunoassay (CLIA), electrochemiluminescence immunoassay (ECLIA), fluorescent antibody assay (FA), radioimmunoassay (RIA), Western blotting method (WB), immunoblotting method, and the like. In any of these assay methods, the protein can be detected or quantified by using VasSF and/or anti-APOA2 antibody.

In the testing method of the present invention, the quantification may be performed by creating a calibration curve based on the relationship between the measured label intensity (for example, absorbance, enzyme label intensity, fluorescence intensity, ultraviolet intensity, radiation intensity, or the like) and the amount (for example, concentration) of the No. 1 APOA2-like protein and/or the No. 2 APOA2-like protein, and performing the quantification based on the calibration curve (for example, by comparison).

According to the present invention, a method for diagnosing vasculitis can also be provided, including the above-described testing method for a disease activity of vasculitis.

According to the present invention, a method for treating and/or preventing vasculitis or COVID-19 can also be provided, including
the testing method of the present invention, and
at least one step selected from the group consisting of a step of subjecting a subject to a treatment according to a diseased state of vasculitis or an exacerbation risk of COVID-19 determined by the testing method, and a step of administering a therapeutic agent or prophylactic agent for vasculitis or COVID-19 according to a diseased state of vasculitis or an exacerbation risk of COVID-19 determined by the testing method to a subject.

### <<Testing kit>>

According to another aspect of the present invention, there are also provided a testing kit for a diseased state of vasculitis and a testing kit for an exacerbation risk of COVID-19, the testing kit including a single chain variable region fragment consisting of an amino acid sequence set forth in SEQ ID NO: 1 or an anti-apolipoprotein A2 antibody. The testing kit is preferably used for the testing method of the present invention mentioned above, and is more preferably a POC (Point of care) kit.

According to still another aspect of the present invention, there is provided a companion diagnostic agent for a diseased state of vasculitis or a companion diagnostic agent for an exacerbation risk of COVID-19 used in the testing method of the present invention described above, the companion diagnostic agent containing a single chain variable region fragment consisting of an amino acid sequence set forth in SEQ ID NO: 1 or an anti-apolipoprotein A2 antibody.

The testing kit and the companion diagnostic agent according to these aspects may include, for example, the following:
(1) a microplate immobilized with VasSF or an anti-APOA2 antibody;
(2) a reaction buffer;
(3) an enzyme-labeled antibody;
(4) an enzyme substrate solution;
(5) a washing agent (any buffer, surfactant, or the like);
(6) a reaction stop solution;
(7) a standard buffer (any buffer or the like); and
(8) a preparation of a No. 1 APOA2-like protein (VAP2) and a No. 2 APOA2-like protein.

The testing kit and the companion diagnostic agent according to these aspects preferably contain a protein preservation buffer containing BSA for the purpose of preventing protein adsorption.

In the present specification, the "companion diagnostic agent" means, for example, a diagnostic agent to be used in a test which is performed before actually starting a treatment, administration of a drug, or the like, in order to predict the effect or risk of a treatment to be performed, the effect of a medicine (a therapeutic agent, a prophylactic agent, or the like) to be administered, the risk of side effects, an appropriate dosage, and the like, for individual patients with vasculitis or COVID-19 according to the diseased state of vasculitis or the exacerbation risk of COVID-19 determined by the testing method of the present invention or the like. The above-mentioned companion diagnostic agent may be used to select a treatment based on the diseased state of vasculitis or an exacerbation risk of COVID-19 determined using the testing method of the present invention mentioned above, or may be used to select a therapeutic agent or prophylactic agent for vasculitis based on the diseased state or a therapeutic agent or prophylactic agent for COVID-19 based on the exacerbation risk.

### EXAMPLES

Hereinafter, the present invention will be described in detail using Examples, but the present invention is not limited to the following Examples. All of the following experiments were approved by the Ethics Committee of Chiba University. In addition, all serum samples were collected from humans after informed consent was obtained from the donors and their willingness was confirmed. Patient specimens were handled and analyzed by coding without personal information.

### [Example 1: Preparation of VasSF and anti-APOA2 antibody]

"VasSF" (comprising an amino acid sequence (SEQ ID NO: 1) encoded by DNA comprising a base sequence of SEQ ID NO: 2 (CDS)), which is a single chain variable region fragment (ScFv), was prepared by the method described in Patent Literature 1. As the anti-apolipoprotein A2 antibody, Anti-apolipoprotein A2, rabbit antibody was purchased from Sigma (cat. no.: SAB1410671) and used in the following experiments.

### [Example 2: Quantification of binding molecules of VasSF and anti-APOA2 antibody]

Quantification of the No. 1 APOA2-like protein (VAP2) and the No. 2 APOA2-like protein, which are molecules to which VasSF and the anti-APOA2 antibody bind, was performed by the following method. That is, albumin and IgG were removed from 5 mL of frozen serum collected from the subject, and binding to VasSF and anti-APOA2 antibody was detected by Western blotting method. The bands thus detected were quantified by imaging signal method and score method. The molecular weight of the No. 1 APOA2-like protein was about 24 kDa and that of the No. 2 APOA2-like protein was about 19/17 kDa.

### [Example 3: Signal analysis of VasSF binding molecules (No. 1 APOA2-like proteins) in serum samples collected from AAV patients]

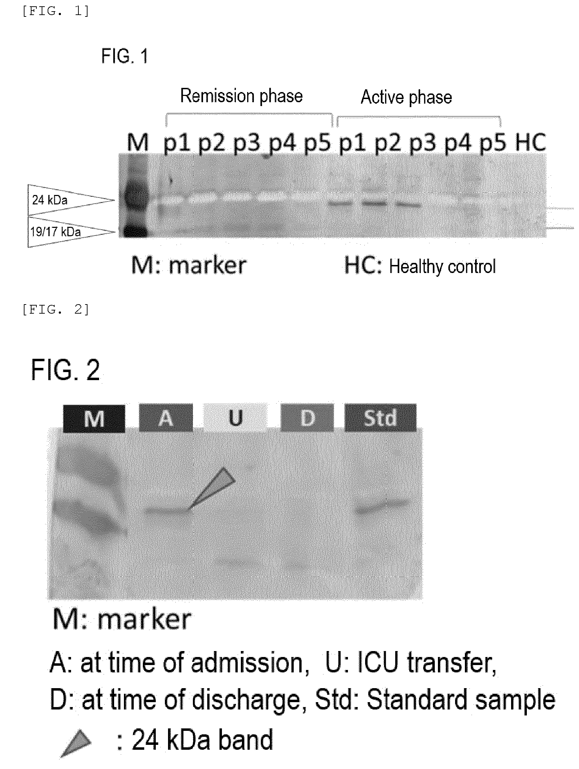

Signal analysis of VasSF binding molecules was performed by Western blotting method using serum samples collected from five patients with ANCA-related vasculitis (AAV) and one healthy control. The results are shown in Fig. 1.

As shown in Fig. 1, in the serum samples of the active phase ("Active" shown in Fig. 1) of AAV in five patients (p1 to p5), a strong band of a protein having a molecular weight of about 24 kDa (No. 1 APOA2-like protein; VAP2) was observed. On the other hand, in the serum samples of the remission phase ("Remission" shown in Fig. 1) of AAV in the same patients, a strong band of a protein having a molecular weight of about 19/17 kDa (No. 2 APOA2-like protein), which is different from the above-mentioned No. 1 APOA2-like protein, was observed. Further, in the serum sample of the healthy control (HC), neither of the bands of the No. 1 APOA2-like nor the No. 2 APOA2-like protein was observed (zero level). Meanwhile, "M" shown in Fig. 1 is a molecular weight marker.

From the above, it is found that both the No. 1 APOA2-like protein (molecular weight: about 24 kDa) and the No. 2 APOA2-like protein (molecular weight: about 19/17 kDa) are useful as markers of disease activity for AAV, and these proteins can be detected using VasSF.

### [Example 4: Signal analysis (1) of VasSF binding molecules (No. 1 APOA2-like proteins) in serum samples collected from COVID-19 patients]

Signal analysis of VasSF binding molecules was performed using serum samples collected from one COVID-19 patient at different time points (at the time of hospital admission, at the time of ICU transfer, and at the time of hospital discharge). The results are shown in Fig. 2.

As shown in Fig. 2, in the serum samples of the patient in an active phase (at the time of hospital admission; "A" shown in Fig. 2) of COVID-19, a strong band of a protein having a molecular weight of about 24 kDa (No. 1 APOA2-like protein; VAP2) was observed. On the other hand, in the serum samples at the time of ICU transfer ("U" shown in Fig. 2) and at the time of hospital discharge ("D" shown in Fig. 2) of the patients, a strong band of a protein having a molecular weight of about 19/17 kDa (No. 2 APOA2-like protein), which is different from the above-mentioned No. 1 APOA2-like protein, was observed. Meanwhile, "M" and "Std" shown in Fig. 2 are a molecular weight marker and a standard sample of VAP2, respectively.

From the above, it is found that both the No. 1 APOA2-like protein (molecular weight: about 24 kDa) and the No. 2 APOA2-like protein (molecular weight: about 19/17 kDa) are useful as markers of disease activity for COVID-19, and these proteins can be detected using VasSF.

### [Example 5: Relationship between signal value of VasSF binding molecules (No. 1 APOA2-like proteins) in serum samples of AAV patients and disease activity]

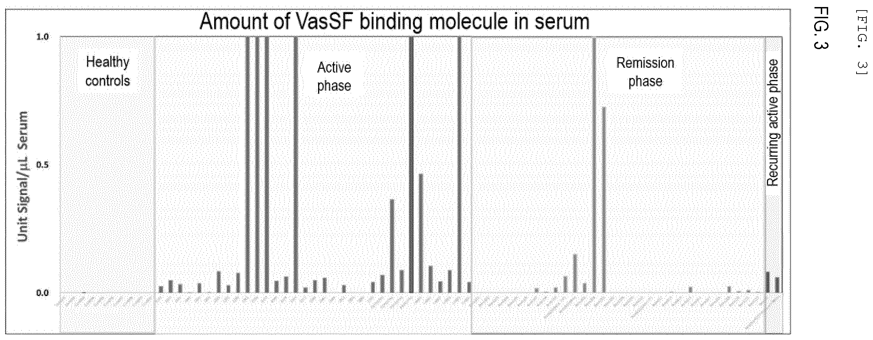

For 67 specimens including 34 specimens of paired sera of the active phase and the remission phase in 17 patients with ANCA-related vasculitis (AAV) and 10 specimens of healthy controls, the relationship between the signal value of VasSF binding molecules and the disease activity of AAV was examined. The results are shown in Fig. 3.

As shown in Fig. 3, in the serum samples from AAV in active phase, the signal value of the VasSF binding molecule (No. 1 APOA2-like protein; VAP2) showed a high level. On the other hand, On the other hand, serum samples from AAV in remission phase and from healthy controls showed low signal values for VasSF binding molecules (No. 1 APOA2-like protein; VAP2) .

Also from the above, it is found that the No. 1 APOA2-like protein (VAP2) is useful as a marker of disease activity for AAV.

### [Example 6: Relationship between signal value of VasSF binding molecules (No. 1 APOA2-like proteins) in serum samples of COVID-19 patients and ]

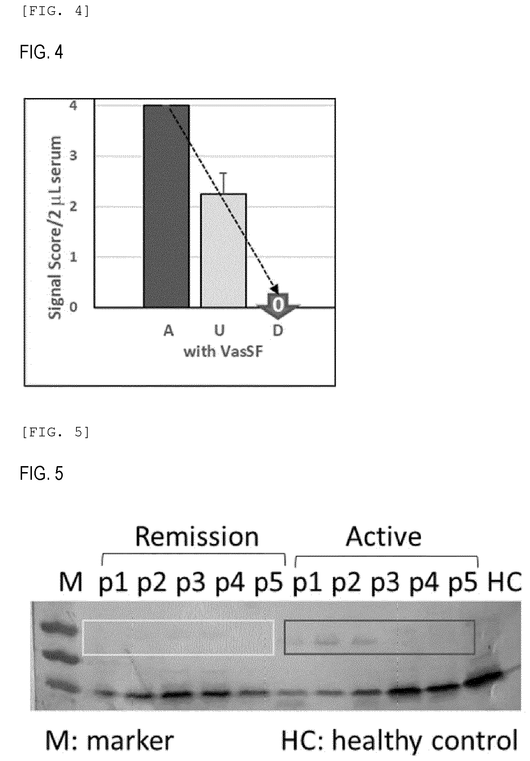

For serum samples collected from 65 COVID-19 patients at different time points (at the time of hospital admission, at the time of ICU transfer, and at the time of hospital discharge), the relationship between the signal value of the VasSF binding molecules and the disease activity of COVID-19 was examined. The results are shown in Fig. 4. Meanwhile, the values of the bar graph and the error bars shown in Fig. 4 show mean ± standard error (SE).

As shown in Fig. 4, in the serum samples of the patients in an active phase (at the time of hospital admission; "A" shown in Fig. 4) of COVID-19, the signal value of the VasSF binding molecule (No. 1 APOA2-like protein; VAP2) showed the highest level (signal score: 4/2 µL serum). On the other hand, this signal value was greatly decreased in the samples at the time of ICU transfer ("U" shown in Fig. 4), and the signal value was a detection limit or lower (signal score was zero) in the samples at the time of hospital discharge ("D" shown in Fig. 4) in the same manner as the healthy controls. Meanwhile, the signal scores of hospital discharge after death and the signal scores at the time of hospital discharge of patients transferred to another hospital immediately after admission were kept at a high level, which were the same as at the time of hospital admission (these results are not included in Fig. 4) in this experiment.

Also from the above, it is found that the No. 1 APOA2-like protein (VAP2) is useful as a marker of disease activity for COVID-19.

### [Example 7: Confirmation of presence of anti-APOA2 antibody binding molecules in serum samples of AAV patients]

In order to confirm whether a VasSF-bound target molecule (No. 1 APOA2-like protein; VAP2) in the experiment using the serum samples collected from the AAV patients described above could also be a target of the anti-APOA2 antibody, signal analysis was performed by the same Western blotting method as in Example 3 above using the anti-APOA2 antibody. The results are shown in Fig. 5.

As shown in Fig. 5, in the serum samples of the active phase ("Active" shown in Fig. 5) of AAV in five patients (p1 to p5), a strong band of a protein (No. 1 APOA2-like protein; VAP2) having a molecular weight of about 24 kDa was observed even in the case of using an anti-APOA2 antibody. On the other hand, in the serum samples of the remission phase ("Remission" shown in Fig. 5) of AAV in the same patients and the serum samples from the healthy controls (HC), a band of the above-mentioned No. 1 APOA2-like protein indicating binding to the anti-AOA2 antibody was not observed (zero level).

From the above, it is found that the No. 1 APOA2-like protein (VAP2) useful as a marker of disease activity for AAV can also be detected using the anti-APOA2 antibody.

### [Example 8: Confirmation of presence of anti-APOA2 antibody binding molecules in serum samples of COVID-19 patients]

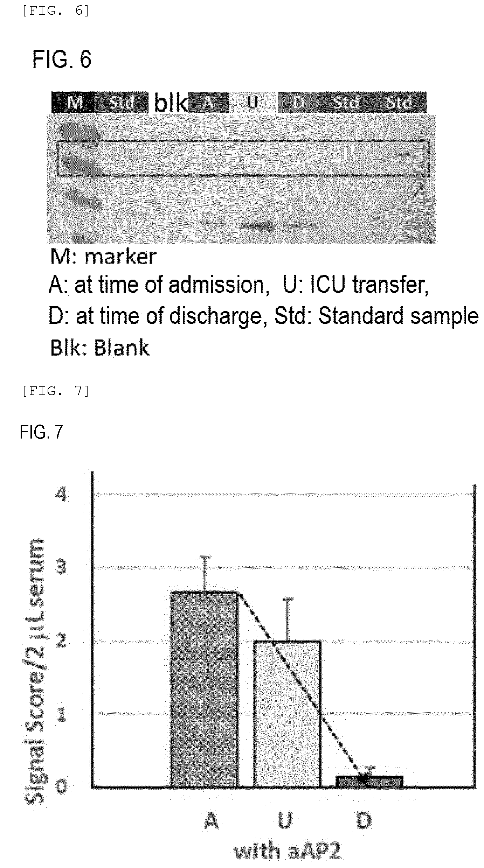

In the experiment using the serum samples collected from the COVID-19 patients described above, in order to confirm whether a VasSF-bound target molecule (No. 1 APOA2-like protein; VAP2) could also be a target of the anti-APOA2 antibody, signal analysis was performed by the same Western blotting method as in Example 4 above using the anti-APOA2 antibody. The results are shown in Fig. 6.

As shown in Fig. 6, in the serum samples of the patients in an active phase (at the time of hospital admission; "A" shown in Fig. 6) of COVID-19, a strong band of a protein (No. 1 APOA2-like protein; VAP2) having a molecular weight of about 24 kDa was observed even in the case of using an anti-APOA2 antibody. On the other hand, in the serum samples at the time of ICU transfer ("U" shown in Fig. 6) and at the time of hospital discharge ("D" shown in Fig. 6) of the patients, a band of the above-mentioned No. 1 APOA2-like protein was not observed (zero level).

From the above, it is found that the No. 1 APOA2-like protein (VAP2) useful as a marker of disease activity for COVID-19 can also be detected using the anti-APOA2 antibody.

### [Example 9: Relationship between signal value of anti-APOA2 antibody binding molecules (No. 1 APOA2-like proteins) in serum samples of COVID-19 patients and disease activity]

For serum samples collected from 65 COVID-19 patients at different time points (at the time of hospital admission, at the time of ICU transfer, and at the time of hospital discharge), the relationship between the signal value of the anti-APOA2 antibody binding molecules and the disease activity of COVID-19 was examined by the same method as in Example 6. The results are shown in Fig. 7. Meanwhile, the values of the bar graph and the error bars shown in Fig. 7 show mean ± standard error (SE).

As shown in Fig. 7, in the serum samples of the patients in an active phase (at the time of hospital admission; "A" shown in Fig. 7) of COVID-19, the signal value of the anti-APOA2 antibody binding molecule (No. 1 APOA2-like protein; VAP2) showed a high level. On the other hand, this signal value was greatly decreased in the samples at the time of ICU transfer ("U" shown in Fig. 7), and the signal value was decreased to the level that the signal score was almost zero at the time of hospital discharge ("D" shown in Fig. 7). Meanwhile, the signal scores of hospital discharge after death and the signal scores at the time of hospital discharge of patients transferred to another hospital immediately after admission were kept at a high level, which were the same as at the time of hospital admission (these results are not included in Fig. 7) in this experiment.

Also from the above, it is found that the No. 1 APOA2-like protein (VAP2) useful as a marker of disease activity for COVID-19 can also be detected using the anti-APOA2 antibody.

### [Example 10: Signal analysis of VasSF binding molecules (No. 1 APOA2-like proteins) in serum samples collected from Kawasaki disease patients]

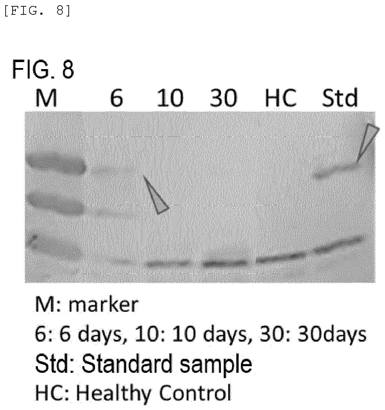

Signal analysis of VasSF binding molecules was performed using serum samples collected from one pediatric patient with Kawasaki disease at different time points (Days 6, 10, and 30 from onset). The results are shown in Fig. 8.

As shown in Fig. 8, in the serum samples of the patient in an active phase (acute phase; Day 6 from onset) of Kawasaki disease, a strong band of a protein (No. 1 APOA2-like protein; VAP2) having a molecular weight of about 24 kDa was observed. On the other hand, in the serum samples collected after the patient's condition stabilized with treatment (days 10 and 30 from onset), a band of the above-mentioned No. 1 APOA2-like protein was not observed (zero level). Meanwhile, "M", "Std" and "HC" shown in Fig. 8 are a molecular weight marker, a standard sample of VAP2, and a sample from healthy subject, respectively.

From the above, it is found that the No. 1 APOA2-like protein (VAP2) is useful as a marker of disease activity for Kawasaki disease, and the protein can be detected using VasSF.

### [Example 11: Signal analysis (2) of VasSF binding molecules (No. 1 APOA2-like proteins) in serum samples collected from COVID-19 patients]

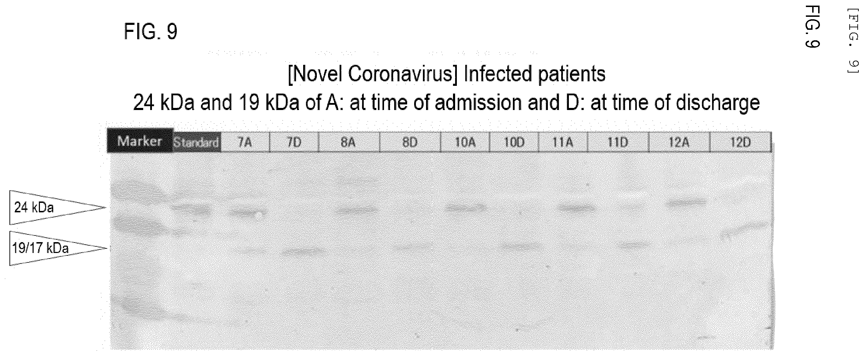

Signal analysis of VasSF binding molecules was performed by the same method as in Example 4 above using serum samples collected from five COVID-19 patients at different time points (at the time of hospital admission and at the time of hospital discharge). The results are shown in Fig. 9.

As shown in Fig. 9, in the serum samples of the patients in an active phase (at the time of hospital admission; "A" shown in Fig. 9) of COVID-19, similarly to Example 4, a strong band of a protein (No. 1 APOA2-like protein; VAP2) having a molecular weight of about 24 kDa was observed. On the other hand, in the serum samples of the patients at the time of hospital discharge ("D" shown in Fig. 9), still similarly to Example 4, a band of the above-mentioned No. 1 APOA2-like protein was not observed (zero level). However, in the serum samples of the patients at the time of hospital discharge ("D" shown in Fig. 9), a strong band of a protein having a molecular weight of about 19/17 kDa (No. 2 APOA2-like protein), which is different from the above-mentioned No. 1 APOA2-like protein, was observed.

Also from the above, it is found that both the No. 1 APOA2-like protein (molecular weight: about 24 kDa) and the No. 2 APOA2-like protein (molecular weight: about 19/17 kDa) are useful as markers of disease activity for COVID-19, and the proteins can also be detected using VasSF.

### [Example 12: Two-dimensional electrophoresis and two-dimensional Western blotting using HRP-VasSF monomer and HRP-anti-APOA2 antibody]

AG(-) proteins with albumin and IgG removed from the serum of one patient in an active phase and one patient in a remission phase of ANCA-related vasculitis (AAV) were prepared and each subjected to two-dimensional electrophoresis (to 10 µg/lane) under non-reducing conditions. According to two-dimensional electrophoresis, proteins can be separated based on molecular weight and isoelectric point. At this time, Precision Plus stained protein standard (manufactured by Bio-Rad Laboratories, Inc.) was used as a molecular weight marker.

After transferring the proteins after two-dimensional electrophoresis to a PVDF membrane, all the proteins on the membrane were visualized using SYPRO Ruby blot stain (manufactured by Thermo Fisher Scientific Inc.). The membrane was then treated with an HRP-conjugated VasSF monomer or an HRP-conjugated anti-APOA2 antibody and further treated with a chemiluminescent substrate. Chemiluminescent images were taken using LAS-3000 (manufactured by Fujifilm Corporation).

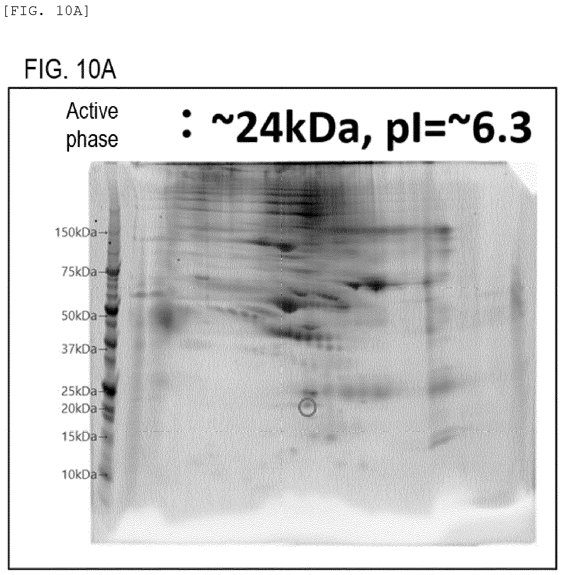

Fig. 10A is a photograph showing the results of two-dimensional electrophoresis performed using serum of a patient in an active phase and treated with VasSF. As shown in Fig. 10A, it was confirmed that a protein having a molecular weight of about 24 kDa and an isoelectric point (pI) of about 6.3 (No. 1 APOA2-like protein) was present in the serum of the patient in an active phase. The same results were obtained when an anti-APOA2 antibody was used instead of VasSF.

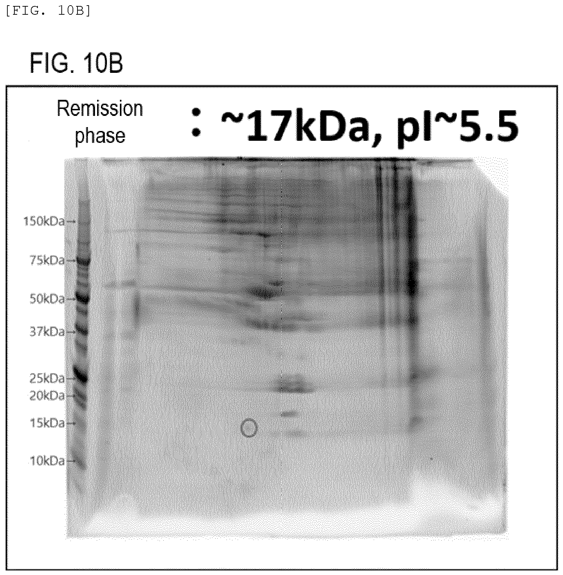

Fig. 10B is a photograph showing the results of two-dimensional electrophoresis performed using serum of a patient in a remission phase and treated with VasSF. As shown in Fig. 10B, it was confirmed that a protein having a molecular weight of about 17 kDa and an isoelectric point (pI) of about 5.5 (No. 2 APOA2-like protein) was present in the serum of the patient in a remission phase. The same results were obtained when an anti-APOA2 antibody was used instead of VasSF.

Also from the above, it is found that both the No. 1 APOA2-like protein (molecular weight: about 24 kDa, isoelectric point: about 6.3) and the No. 2 APOA2-like protein (molecular weight: about 17 kDa, isoelectric point: about 5.5) are useful as markers of disease activity for COVID-19, and these proteins can also be detected using VasSF.

From the above-described series of results, it was shown that the No. 1 APOA2-like protein (VAP2) that is present at a high level in the serum of a patient in an active phase of vasculitis and a disease accompanied with vascular inflammation such as ANCA-related vasculitis, COVID-19, and Kawasaki disease and disappears after the symptoms are alleviated is useful as a marker of disease activity for these diseases. It was also shown that the No. 2 APOA2-like protein, which is not found in the serum in an active phase of the disease and is present at a high level in the serum after the symptoms are alleviated, is also useful as a marker of disease activity for each of the above diseases.

It can be said that the VasSF and the anti-APOA2 antibody capable of detecting and quantifying the No. 1 APOA2-like protein and the No. 2 APOA2-like protein that increase and decrease with the passage of each of the above diseases are also useful as a companion diagnostic agent for a diseased state of vasculitis used in a testing method for a diseased state of vasculitis using the above protein as a marker.

Since there is a report that vasculitis is involved in the exacerbation of COVID-19, it can be said that the No. 1 APOA2-like protein (VAP2) and the No. 2 APOA2-like protein are also useful as testing markers for the exacerbation risk of COVID-19, as with vasculitis such as Kawasaki disease described above.

### [Sequence listing free text]

SEQ ID NOs in the Sequence Listing of the present specification show the following sequences.
[SEQ ID NO: 1]
   Amino acid sequence of one (VasSF) of single chain variable region fragments (ScFv) binding the No. 1 APOA2-like protein and the No. 2 APOA2-like protein
[SEQ ID NO: 2]
   Base sequence of DNA (CDS; including a stop codon) encoding VasSF

## Claims

1. A method for testing a diseased state of vasculitis, the method comprising:
detecting or quantifying, in a blood sample collected from a subject,
(1) a No. 1 APOA2-like protein that causes an antigen-antibody reaction with a single chain variable region fragment consisting of an amino acid sequence set forth in SEQ ID NO: 1 and has a molecular weight of 23 to 25 kDa, and/or
(2) a No. 2 APOA2-like protein that causes an antigen-antibody reaction with a single chain variable region fragment consisting of an amino acid sequence set forth in SEQ ID NO: 1 and has a molecular weight of 16 to 20 kDa.

2. The method according to claim 1, wherein the vasculitis is vasculitis associated with COVID-19, ANCA-related vasculitis, or Kawasaki disease.

3. The method according to claim 1 or 2, wherein an isoelectric point (pI) of the No. 1 APOA2-like protein is 6.0 to 6.6, and an isoelectric point (pI) of the No. 2 APOA2-like protein is 5.2 to 5.8.

4. A method for testing an exacerbation risk of COVID-19, the method comprising:
detecting or quantifying, in a blood sample collected from a subject,
(1) a No. 1 APOA2-like protein that causes an antigen-antibody reaction with a single chain variable region fragment consisting of an amino acid sequence set forth in SEQ ID NO: 1 and has a molecular weight of 23 to 25 kDa, and/or
(2) a No. 2 APOA2-like protein that causes an antigen-antibody reaction with a single chain variable region fragment consisting of an amino acid sequence set forth in SEQ ID NO: 1 and has a molecular weight of 16 to 20 kDa.

5. The method according to claim 4, wherein an isoelectric point (pI) of the No. 1 APOA2-like protein is 6.0 to 6.6, and an isoelectric point (pI) of the No. 2 APOA2-like protein is 5.2 to 5.8.

6. The method according to claim 1 or 4, comprising detecting or quantifying the No. 1 APOA2-like protein and the No. 2 APOA2-like protein.

7. The method according to claim 1 or 4, wherein the blood sample is plasma or serum.

8. A testing kit for a diseased state of vasculitis, the testing kit comprising a single chain variable region fragment consisting of an amino acid sequence set forth in SEQ ID NO: 1 or an anti-apolipoprotein A2 antibody.

9. The testing kit according to claim 8, which is used for the method according to claim 1.

10. A testing kit for an exacerbation risk of COVID-19, the testing kit comprising a single chain variable region fragment consisting of an amino acid sequence set forth in SEQ ID NO: 1 or an anti-apolipoprotein A2 antibody.

11. The testing kit according to claim 10, which is used for the method according to claim 4.

12. A companion diagnostic agent for a diseased state of vasculitis used in the method according to claim 1, the companion diagnostic agent comprising a single chain variable region fragment consisting of an amino acid sequence set forth in SEQ ID NO: 1 or an anti-apolipoprotein A2 antibody.

13. The companion diagnostic agent according to claim 12, which is used for selecting a treatment according to a diseased state of vasculitis determined using the method according to claim 1 and/or selecting a therapeutic agent or prophylactic agent for vasculitis according to the diseased state.

14. A companion diagnostic agent for an exacerbation risk of COVID-19 used in the method according to claim 4, the companion diagnostic agent comprising a single chain variable region fragment consisting of an amino acid sequence set forth in SEQ ID NO: 1 or an anti-apolipoprotein A2 antibody.

15. The companion diagnostic agent according to claim 14, which is used for selecting a treatment according to an exacerbation risk of COVID-19 determined using the method according to claim 4 and/or selecting a therapeutic agent or prophylactic agent for vasculitis according to the diseased state.

16. A testing marker for an exacerbation risk of COVID-19, the testing marker comprising (1) a No. 1 APOA2-like protein that causes an antigen-antibody reaction with a single chain variable region fragment consisting of an amino acid sequence set forth in SEQ ID NO: 1 and has a molecular weight of 23 to 25 kDa.

17. The testing marker according to claim 16, wherein an isoelectric point (pI) of the No. 1 APOA2-like protein is 6.0 to 6.6.

18. A testing marker for an exacerbation risk of COVID-19, the testing marker comprising (2) a No. 2 APOA2-like protein that causes an antigen-antibody reaction with a single chain variable region fragment consisting of an amino acid sequence set forth in SEQ ID NO: 1 and has a molecular weight of 16 to 20 kDa.

19. The testing marker according to claim 18, wherein an isoelectric point (pI) of the No. 2 APOA2-like protein is 5.2 to 5.8.
